# EUROPEAN PATENT APPLICATION

(11) **EP 3 995 091 A1**
(43) Date of publication of application: **11.05.2022**
(21) Application number: 20836171.7
(22) Date of filing: 28.06.2020
(51) Int. Cl.: A61B 17/42, A61B 1/05, A61M 1/00

(54) **DIRECT-VISION ABORTION UTERINE CURETTAGE DEVICE AND SYSTEM**

(30) Priority: 06.07.2019 CN 201910612451
(71) Applicant: Zhou, Xing, Guangzhou, Guangdong 510663 (CN)
(72) Inventor: Zhou, Xing, Guangzhou, Guangdong 510663 (CN)
(74) Representative: Boult Wade Tennant LLP
(86) International application number: PCT/CN2020/098463
(87) International publication number: WO 2021/004302

(57) **Abstract**

A direct-vision abortion uterine curettage device (100) and a direct-vision abortion uterine curettage system (900) are provided. The direct-vision abortion uterine curettage device (100) includes a uterine curettage mechanism (1), an observation mechanism (2), a circuit (3), a negative pressure suction mechanism (4) and an operating rod (5). A diagnostic curette (11) of the uterine curettage mechanism (1) is arranged at front ends of the operating rod (5) and a camera (22-1) and is in a visual field of the camera (22-1). A technical solution of the camera (22-1) being mounted at the rear may guarantee that the diagnostic curette (11) is completely within a range of the visual field of the camera (22-1), so that an operation procedure may be fully visible without any observation blind areas. At the same time, there will be no operation blind spots due to the diagnostic curette (11) being blocked by a height of a lens module (22) itself. The embryo tissue implanted at any position of the uterus, especially near the oviduct orifice, may be completely removed, i.e. the diagnostic curette (11) may also easily remove the embryo tissue at the implantation position of the uterine fundus, and the clinical use process is safer and more efficient. The direct-vision abortion uterine curettage system (900) includes the direct-vision abortion uterine curettage device (100) and may perform surgical operations under real-time display of a display system (102). Therefore, an abortion operation procedure is very accurate, safe and efficient.

## Description

### FIELD OF THE INVENTION

The present invention relates to an endoscopic surgical instrument, and particularly relates to a direct-vision abortion uterine curettage device and system used in an endoscopic operation.

### BACKGROUND OF THE INVENTION

An abortion operation refers to termination of pregnancy by an operation method. Abortion operation methods usually include negative pressure suction and forceps curettage. A suction pipe is used to suck the embryo tissue out of the uterus or surgical instruments such as oval forceps are used to take the embryo tissue out of the uterus, so as to achieve the purpose of termination of pregnancy. Therefore, the core of the abortion operation lies in two aspects: firstly, the embryo tissue needs to be removed completely to prevent incomplete abortion; and secondly, accidental injuries to the uterus, such as accidental perforation of uterus, during the operation procedure need to be prevented.

The abortion operation needs to be performed in the uterus, and a traditional abortion operation often relies on the experience and feeling of doctors, so it is prone to various complications. With the development of the endoscopic technology, more and more operations are completed under an endoscope, and the abortion operation is also performed under endoscopic direct vision gradually.

In a direct-vision abortion device of the prior art, a lens module is arranged at the most front end of the direct-vision abortion device. After the embryo implantation tissue is found through a camera, a negative pressure suction port located at the rear end of the camera is used to suck the implanted embryo tissue, and then, peeling of the implanted embryo is completed through repeated insertion and extraction of an abortion pipe. Since the camera is located at the most front end, during the clinical operation of the direct-vision abortion device in the prior art, the specific process of peeling the embryo from the implantation position is still invisible, and there is a clinical risk of incomplete abortion. In addition, since the lens module has a certain height and there is a distance of at least 8 mm to the negative pressure suction port, the direct-vision abortion device in the prior art is very difficult to remove the implanted embryo near the oviduct orifice, namely near the uterine fundus. Moreover, if the insertion and extraction depth of the abortion pipe is too large, it is easy to cause accidental injuries to the uterine fundus, and even cause a serious consequence of perforation of uterus.

Therefore, it is necessary to further improve the existing direct-vision abortion device to guarantee that the separation process of the embryo from the implantation position is fully visible, avoid the risk of incomplete abortion, better avoid accidental injuries to the uterus, and improve the accuracy and efficiency of the abortion procedure.

### SUMMARY

In a direct-vision abortion uterine curettage device and system of the present invention, through an observation system, especially the design of the observation system mounted at the rear, a uterine curettage mechanism is mounted at the front, thereby guaranteeing that in an embryo removing procedure, there are no operation blind spots, so as to avoid the condition of incomplete abortion. Moreover, the design of the rear-mounted observation system may guarantee that the whole process of an observation operation may be in a visual field of observation, thereby effectively avoiding an accidental injury to the uterus. The clinical use process of the direct-vision abortion uterine curettage device and system of the present invention is safer and more efficient.

The direct-vision abortion uterine curettage device and system of the present invention have the characteristics that: the direct-vision abortion uterine curettage device 100 includes a uterine curettage mechanism 1, an observation mechanism 2, a circuit 3, a negative pressure suction mechanism 4 and an operating rod 5;
A. the uterine curettage mechanism 1 includes at least one diagnostic curette 11, and the diagnostic curette 11 is arranged at a front end of the operating rod 5 and is in a visual field of the observation mechanism 2;
B. the observation mechanism 2 includes an illumination module 21, a lens module 22 and a signal processing module 23, and images and videos observed by the lens module 22 in a light field of the illumination module 21 are processed by the signal processing module 23, and then output by the circuit 3;
C. the negative pressure suction mechanism 4 includes a suction inlet 41, a suction outlet 42 and a suction channel 43, the suction inlet 41 is arranged at the front end of the operating rod 5, the suction outlet 42 of the negative pressure suction mechanism 4 is arranged at a rear end of the operating rod 5, and the suction channel 43 is arranged in the operating rod 5; and
D. the rear end 5-2 of the operating rod 5 is provided with a handle 52, the front end 5-1 of the operating rod 5 is provided with the uterine curettage mechanism 1 and the observation mechanism 2, and the circuit 3 and the negative pressure suction mechanism 4 are located in a pipe body 5-3 of the operating rod 5.

The diagnostic curette 11 is arranged in a visual field of the observation mechanism 2. During a clinical operation, under the action of the lens module 22 of the observation mechanism 2, the whole operation procedure of an abortion operation may be performed under the condition of direct vision, so that whether the embryo tissue is completely removed during the operation procedure may be clearly observed, whether an accidental injury to the uterus is caused during the operation procedure may also be observed, and the clinical use process is very safe and reliable. In addition, the uterine curettage mechanism 1 and the negative pressure suction mechanism 4 may be designed as relatively independent mechanisms, so that on the premise of guaranteeing the curettage effect of the uterine curettage mechanism 1 on the implanted embryo, the negative pressure suction mechanism 4 may also very effectively suck and discharge the curetted tissue and the generated tissue fluids such as watery blood quickly out of the body.

A camera 22-1 of the lens module 22 is arranged at a rear end of the diagnostic curette 11, and the diagnostic curette 11 is in a visual field of the camera 22-1.

The technical solution of the camera 22-1 being mounted at the rear may guarantee that the diagnostic curette 11 is completely within a range of the visual field of the camera 22-1, and since an observation angle of the camera 22-1 faces the front of an operation area, there will be no observation blind areas during the observation process. As a result, there will be no accidental injuries to the uterus and uterine appendages caused by the failure to observe clearly in time. At the same time, since the diagnostic curette 11 is located in front of the camera 22-1, there will be no operation blind spots due to the diagnostic curette 11 being blocked by a height of the lens module 22 itself. The embryo tissue implanted at any position of the uterus, especially near the oviduct orifice, may be completely removed, i.e. the diagnostic curette 11 may also easily remove the embryo tissue at the implantation position of the uterine fundus, and the condition of incomplete abortion may be avoided during the operation procedure. Moreover, since the removal is performed under direct vision, an accidental injury to the uterine fundus due to an excessive surgical action may be avoided, the injury to the uterine fundus may be well avoided, and serious medical accidents such as perforation of uterus may be avoided. Therefore, the technical solution of the camera being mounted at the rear makes the clinical operation procedure of abortion safer and more efficient.

The direct-vision abortion uterine curettage device 100 further includes a flushing mechanism 6; a water outlet 61 of the flushing mechanism 6 is located at the front end of the operating rod 5, a water inlet 62 of the flushing mechanism 6 is located at the rear end of the operating rod 5, and a water inlet pipeline 63 of the flushing mechanism 6 is located in the pipe body 5-3 of the operating rod 5. The flushing mechanism 6 may flush the inside of the uterus during the operation procedure, and may flush the watery blood at the front end of the camera 22-1 in time to guarantee that the visual field of observation is kept clean. At the same time, the flushing mechanism 6 may also flush the diagnostic curette 11 and the uterus wall in time, especially may flush a surgical site in time, so as to observe whether the embryo tissue has been completely removed from the implantation position in time to guarantee the completeness of the embryo tissue during the abortion operation procedure and effectively avoid the condition of incomplete abortion, and the clinical operation procedure is safer.

The pipe body 5-3 of the operating rod 5 is a double-cavity pipe, the circuit 3 is arranged in one cavity pipe, and the other cavity pipe constitutes or is provided with the suction channel 43 of the negative pressure suction mechanism 4. The design of the double-cavity channel may isolate the circuit 3 from fluids such as watery blood and tissue fluids that may be generated during the operation, so as to better avoid potential safety hazards such as short circuit and electric leakage during the use process, and the clinical use process is safer.

The pipe body 5-3 of the operating rod 5 is a three-cavity pipe, the circuit 3 is arranged in the first cavity pipe, the second cavity pipe constitutes or is provided with the suction channel 43 of the negative pressure suction mechanism 4, and the third cavity pipe constitutes or is provided with the water inlet pipeline 63 of the flushing mechanism 6. The design of the three-cavity channel may separate a flushing channel from the suction channel while guaranteeing that the circuit 3 is isolated and protected, so as to better guarantee that no sucked tissue or fluid will return to the uterus during the flushing process, and the use process is more efficient and safer.

Water sprayed from at least one water outlet 61 of the water outlets 61 of the flushing mechanism 6 of the direct-vision abortion uterine curettage device 100 may flush and clean a protective cover 20 of the observation mechanism 2. The flushing mechanism 6 may flush the protective cover 20 of the observation mechanism 2 in time, so as to guarantee the observation effect of the observation mechanism 2.

The flushing mechanism 6 of the direct-vision abortion uterine curettage device 100 is provided with a flushing switch 64 for controlling flushing water. The flushing switch 64 may start and stop the flushing mechanism 6, and may control the water volume and flushing pressure of the flushing water. During the clinical use process, clinicians may start or stop the flushing mechanism 6 according to actual needs of the operation procedure, and select the appropriate volume and pressure of the flushing water according to needs, and the clinical use process is more convenient.

The flushing switch 64 is arranged on the handle 52 of the operating rod 5. By arranging the flushing switch 64 on the handle 52, the medical staff may realize one-handed operation during clinical use, and the use process is more convenient.

The negative pressure suction mechanism 4 is provided with a negative pressure control switch 44 capable of controlling a negative pressure state. The negative pressure control switch 44 may start or stop the negative pressure suction mechanism 4, and may adjust the negative pressure suction force of the negative pressure suction mechanism 4 according to the volume of the peeled tissue, watery blood and tissue fluids in the uterus during the operation procedure. During clinical use, doctors may conveniently adjust the needed negative pressure suction force, so as to avoid that the peeled tissue may not be sucked out in time due to a too small negative pressure suction force, and avoid accidental injuries to the endometrium and other tissues due to a too large negative pressure suction force.

The negative pressure control switch 44 is arranged on the handle 52 of the operating rod 5. By arranging the negative pressure control switch on the handle 52, the medical staff may realize one-handed operation during clinical use, and the use process is more convenient.

The diagnostic curette 11 of the uterine curettage mechanism 1 is a curette spoon 11-1.

A front end of the curette spoon 11-1 is a blunt end, and a curette hook 11-11 of the curette spoon 11-1 is bent inward. The design of the blunt end may prevent the curette spoon 11-1 from accidentally puncturing the tissue at the front end of the curette spoon 11-1 during the forward moving process, and the clinical use process is safer. The inward bent curette hook 11-11 may conveniently remove the implanted embryo tissue.

The curette spoon 11-1 is made of a transparent medical material. The transparent material may guarantee that the curette spoon 11-1 may not block an observation path of the camera 22-1, thereby guaranteeing that the tissue at the front end of the curette spoon 11-1 may also be observed by the observation mechanism 2, and guaranteeing the whole-process and overall-area observation of the operation procedure.

The diagnostic curette 11 of the uterine curettage mechanism 1 is a curette cup 11-2.

A front end of the curette cup 11-2 is a blunt end, and at least one diagnostic curette strip 11-21 is arranged around the blunt end. The design of the blunt end may guarantee that the curette cup 11-2 may not accidentally puncture the tissue at the front end of the curette cup 11-2 during the forward moving process. During clinical use, the diagnostic curette strip 11-21 may conveniently remove the implanted embryo tissue from the uterus.

The curette cup 11-2 is made of a transparent medical material. The transparent material may guarantee that the curette cup 11-2 may not block the observation path of the camera 22-1, thereby guaranteeing that the tissue at the front end of the curette cup 11-2 may also be observed by the observation mechanism 2, and guaranteeing the whole-process and overall-area observation of the operation procedure.

The diagnostic curette 11 of the uterine curettage mechanism 1 is a curette mesh 11-3. Through the camera 22-1, the condition behind the curette mesh 11-3 may be directly observed from mesh gaps, and even if the curette mesh 11-3 is not made of a transparent material, the whole-process observation of the operation procedure and the panoramic observation of the operation area may be realized.

The curette mesh 11-3 of the diagnostic curette 11 of the uterine curettage mechanism 1 may be compressed in a sheath pipe 5-0.

The curette mesh 11-3 of the diagnostic curette 11 of the uterine curettage mechanism 1 may be compressed in the sheath pipe 5-0, and after the constraint of the sheath pipe 5-0 is removed backward, the curette mesh 11-3 may be completely restored or basically restored to a shape before compression. During clinical use, the curette mesh 11-3 may be folded into the sheath pipe 5-0 and enters the uterus through the cervix, then the sheath pipe 5-0 may be retracted to release the curette mesh 11-3, and the curette mesh 11-3 may be unfolded to remove the embryo tissue.

The curette mesh 11-3 includes at least one diagnostic curette wire 11-31. The curette mesh 11-3 may be formed by winding one diagnostic curette wire 11-31 or superimposing a plurality of diagnostic curette wires 11-31 after being wound. Moreover, the curette mesh 11-3 may be manufactured in any shapes which are not illustrated by the applicant here one by one, but do not depart from the protection scope of the present application.

The diagnostic curette 11 of the uterine curettage mechanism 1 is a woven mesh made of a wire material. The design of the soft woven mesh makes the force applied during the implanted embryo removing process more gentle and even, so that the injury to the uterus is less.

The curette mesh 11-3 is made of a medical elastic material. The curette mesh 11-3 may guarantee the removing effect, and at the same time, during the forward moving process, the elastic action of the medical elastic material may play a good buffering effect, so that the curette mesh 11-3 will not cause an accidental injury to the uterus due to an excessively fast moving speed.

The curette mesh 11-3 is made of medical elastic stainless steel or medical titanium-nickel shape memory alloy. Here, the applicant only lists the above two medical elastic materials. In practical applications, those skilled in the art may also select different medical elastic materials for manufacturing according to needs, and all the medical elastic materials do not depart from the protection scope of the present application.

The curette mesh 11-3 is made of a medical transparent elastic polymer material. The curette mesh 11-3 may also be made of the medical transparent elastic polymer material, so that the observation path of the camera 22-1 may not be blocked while guaranteeing elastic buffering, and the observation effect is better.

The protective cover 20 of the observation mechanism 2 is provided with a coating. A hydrophobic coating may be selected, so that fluids such as watery blood may quickly condense into water droplets on the protective cover 20 and then slide off. A hydrophilic coating may also be selected, so that fluids such as watery blood may quickly form a transparent water film on a surface of the protective cover 20 without blocking the camera.

The circuit 3 is provided with an electrical interface 31 connected with a host.

The direct-vision abortion uterine curettage device 100 further includes an identification system 8. The identification system 8 may prompt the depth of the operating rod 5 into the human body. The identification system 8 may be a graduated scale 81 arranged outside the operating rod 5, or other identification methods may be used. The other identification methods are not specifically illustrated by the applicant here one by one, but do not depart from the protection scope of the present application.

A direct-vision abortion uterine curettage system has the characteristics that: the direct-vision abortion uterine curettage system 900 includes the direct-vision abortion uterine curettage device 100.

The direct-vision abortion uterine curettage system 900 includes the direct-vision abortion uterine curettage device 100, a housing 101, a display system 102, an image processing system 103 and a power supply system 104; the display system 102 is arranged on the housing 101; the image processing system 103 and the power supply system 104 are installed in the housing 101; the display system 102, the image processing system 103 and the power supply system 104 are connected together through the circuit 3; and the suction outlet 42 of the negative pressure suction mechanism 4 of the direct-vision abortion uterine curettage device 100 is connected with a negative pressure suction apparatus of a hospital or a negative pressure pipe of a medical special negative pressure source.

During clinical use, the suction outlet 42 is connected with the negative pressure suction apparatus of the hospital or the negative pressure pipe of the medical special negative pressure source; the power supply system 104 is turned on, the direct-vision abortion uterine curettage system 900 starts to work; the observation mechanism 2 is started, and video signals collected by the observation mechanism 2 are processed by the image processing system 103 and then may be transmitted to the display system 102 in a wired or wireless manner and displayed on the display system 102. Under the observation of the camera 22-1, the direct-vision abortion uterine curettage device 100 enters the uterus through the cervix along the vagina to find the implanted embryo tissue, then the diagnostic curette 11 of the uterine curettage mechanism 1 is used to peel the implanted embryo tissue from the uterus; the negative pressure suction apparatus or the medical special negative pressure source is turned on, and the peeled embryo tissue and accompanying watery blood are sucked through the suction outlet 42 and discharged out of the body until the embryo tissue is completely peeled and sucked and discharged out of the body, thereby completing the abortion operation.

The direct-vision abortion uterine curettage system 900 includes the direct-vision abortion uterine curettage device 100, a housing 101, a display system 102, an image processing system 103, a power supply system 104 and a negative pressure suction apparatus 105; the display system 102 is arranged on the housing 101; the image processing system 103, the power supply system 104 and the negative pressure suction apparatus 105 are installed in the housing 101; the display system 102, the image processing system 103, the power supply system 104 and the negative pressure suction apparatus 105 are connected together through the circuit 3; and the suction outlet 42 of the negative pressure suction mechanism 4 of the direct-vision abortion uterine curettage device 100 is connected with the negative pressure suction apparatus 105.

The direct-vision abortion uterine curettage system 900 directly includes the negative pressure suction apparatus 105, so that the direct-vision abortion uterine curettage system of the present invention may be directly used to perform a direct-vision abortion operation without relying on external negative pressure sources. The direct-vision abortion uterine curettage system of the present invention is not limited by external negative pressure sources, and has wider application scenes.

The direct-vision abortion uterine curettage system 900 includes the direct-vision abortion uterine curettage device 100, a housing 101, a display system 102, an image processing system 103, a power supply system 104 and a flushing system 106; the display system 102 is arranged on the housing 101; the image processing system 103, the power supply system 104 and the flushing system 106 are installed in the housing 101; the display system 102, the image processing system 103, the power supply system 104 and the flushing system 106 are connected together through the circuit 3; the suction outlet 42 of the negative pressure suction mechanism 4 of the direct-vision abortion uterine curettage device 100 is connected with a negative pressure suction apparatus of a hospital or a negative pressure pipe of a medical special negative pressure source; and a water inlet 106-1 of the flushing system 106 is connected with an infusion bottle or bag 7 through a water pipe 106-4, and a water outlet 106-2 of the flushing system 106 is connected with the water inlet 62 of the flushing mechanism 6 of the direct-vision abortion uterine curettage device 100 through the water pipe 106-4.

The direct-vision abortion uterine curettage system 900 includes the flushing system 106, and the flushing system 106 may flush the direct-vision abortion uterine curettage device 100 and surrounding tissues in time during the operation procedure, so as to flush a surgical site in time while guaranteeing that the visual field of observation is kept clean, thereby observing whether the embryo tissue has been completely removed from the implantation position in time to guarantee the completeness of the embryo tissue during the abortion operation procedure and effectively avoid the condition of incomplete abortion. The clinical operation procedure is safer.

The direct-vision abortion uterine curettage system 900 includes the direct-vision abortion uterine curettage device 100, a housing 101, a display system 102, an image processing system 103, a power supply system 104, a negative pressure suction apparatus 105 and a flushing system 106; the display system 102 is arranged on the housing 101; the image processing system 103, the power supply system 104, the negative pressure suction apparatus 105 and the flushing system 106 are installed in the housing 101; the display system 102, the image processing system 103, the power supply system 104, the negative pressure suction apparatus 105 and the flushing system 106 are connected together through the circuit 3; the suction outlet 42 of the negative pressure suction mechanism 4 of the direct-vision abortion uterine curettage device 100 is connected with the negative pressure suction apparatus 105; and a water inlet 106-1 of the flushing system 106 is connected with an infusion bottle or bag 7 through a pipeline 7-1, and a water outlet 106-2 of the flushing system 106 is connected with the water inlet 62 of the flushing mechanism 6 through the pipeline 7-1.

The direct-vision abortion uterine curettage system 900 includes the negative pressure suction apparatus 105 and the flushing system 106, and may complete the whole operation procedure of direct-vision abortion alone without relying on external negative pressure sources and external flushing systems, thereby greatly reducing the limitation and influence of the external environment on the operation procedure.

The flushing system 106 is driven by a peristaltic pump 106-3. The peristaltic pump 106-3 may more accurately control the flow rate and speed of water intake. Of course, in practical applications, those skilled in the art may also use different driving devices to drive the flushing system 106, which does not depart from the protection scope of the present application.

The direct-vision abortion uterine curettage device of the present invention includes the uterine curettage mechanism 1, the observation mechanism 2, the circuit 3, the negative pressure suction mechanism 4 and the operating rod 5. The diagnostic curette 11 of the uterine curettage mechanism 1 is arranged at the front ends of the operating rod 5 and the camera 22-1 and is in the visual field of the camera 22-1. The technical solution of the camera 22-1 being mounted at the rear may guarantee that the diagnostic curette 11 is completely within a range of the visual field of the camera 22-1, so that an operation procedure may be fully visible without any observation blind areas. At the same time, there will be no operation blind spots due to the diagnostic curette 11 being blocked by the height of the lens module 22 itself. The embryo tissue implanted at any position of the uterus, especially near the oviduct orifice, may be completely removed, i.e. the diagnostic curette 11 may also easily remove the embryo tissue at the implantation position of the uterine fundus, and the clinical use process is safer and more efficient. The direct-vision abortion uterine curettage system of the present invention includes the direct-vision abortion uterine curettage device of the present invention and may perform surgical operations under the real-time display of the display system. Therefore, the abortion operation procedure is very accurate, safe and efficient.

### BRIEF DESCRIPTION OF THE DRAWINGS

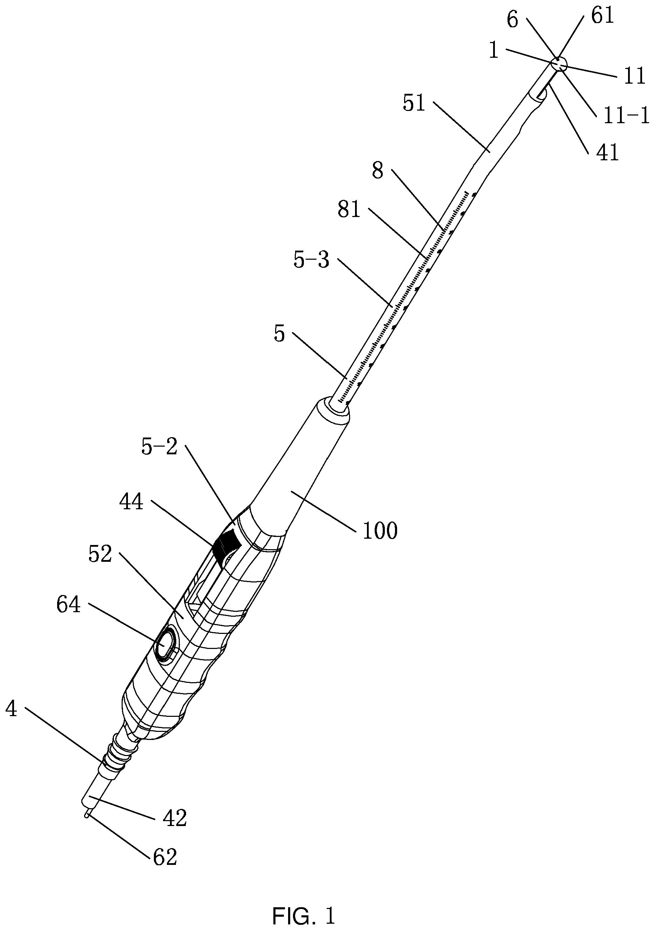

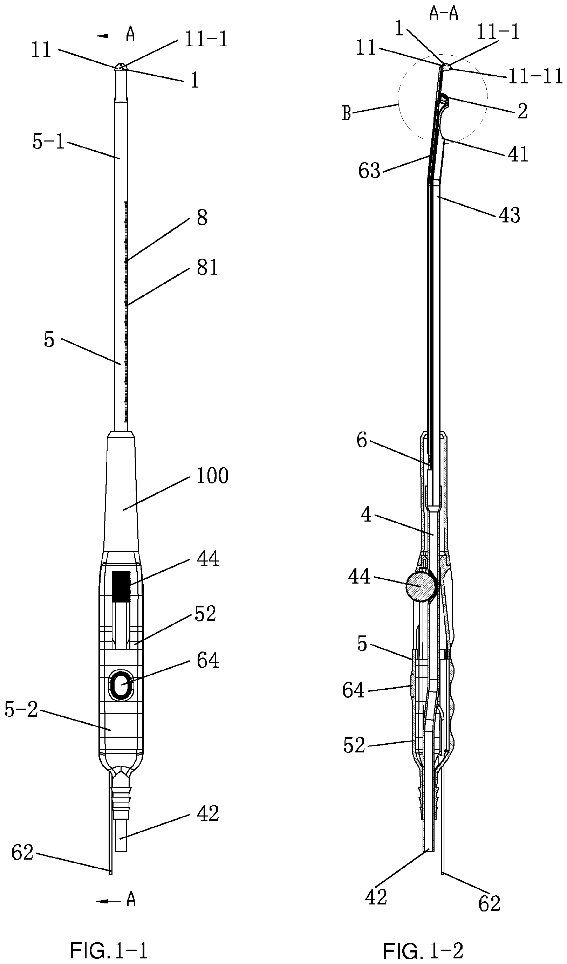

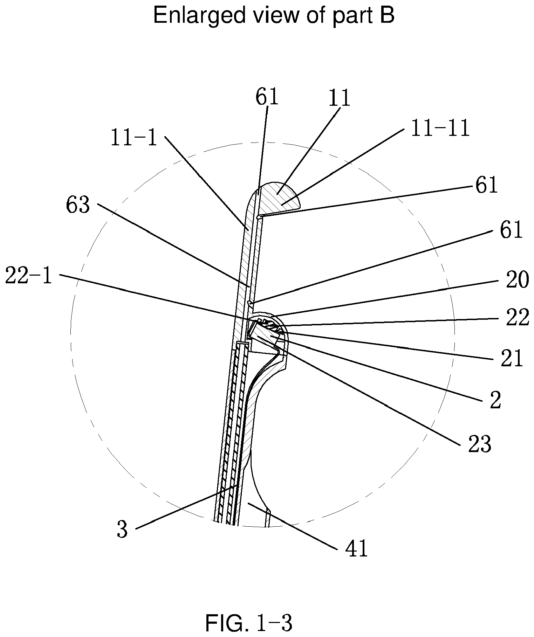

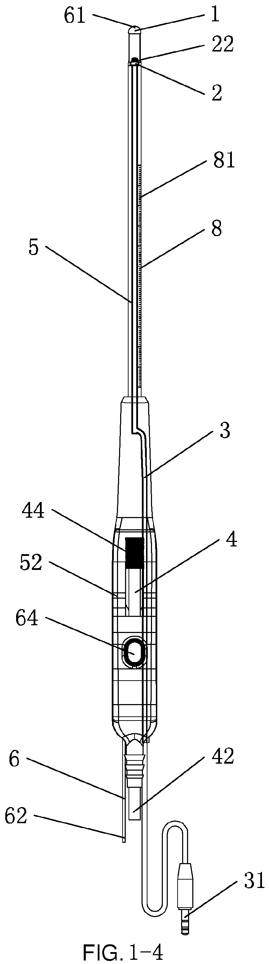

FIG. 1 is a three-dimensional schematic structural view of a direct-vision abortion uterine curettage device of the present invention with a curette spoon.
FIG. 1-1 is a front view of FIG. 1.
FIG. 1-2 is a cross-sectional view taken along line A-A in FIG. 1-1.
FIG. 1-3 is an enlarged view of part B in FIG. 1-2.
FIG. 1-4 is a schematic circuit diagram of FIG. 1.
FIG. 2 is a three-dimensional schematic structural view of a direct-vision abortion uterine curettage device of the present invention with a curette cup.
FIG. 2-1 is a front view of FIG. 2.
FIG. 2-2 is a cross-sectional view taken along line C-C in FIG. 2.

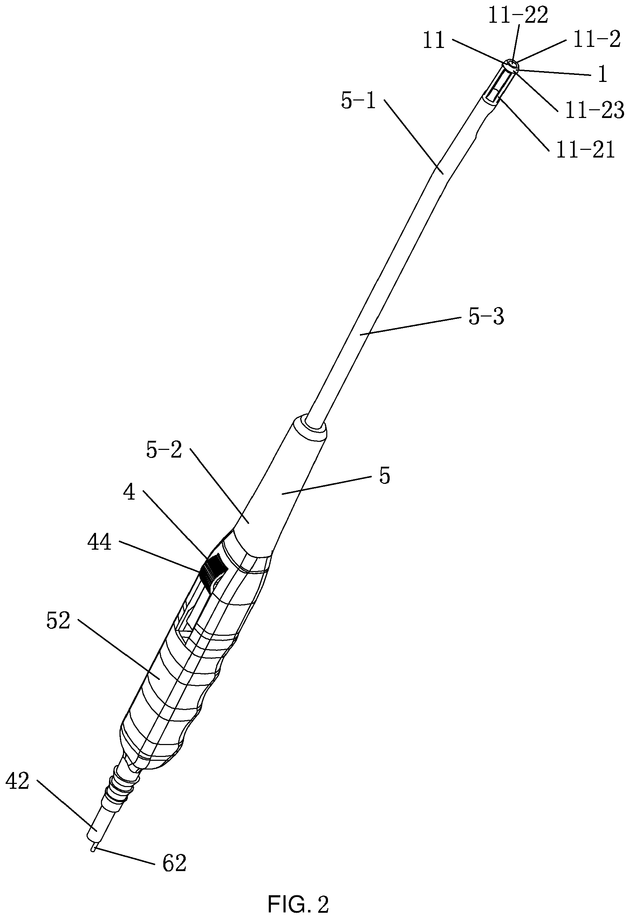

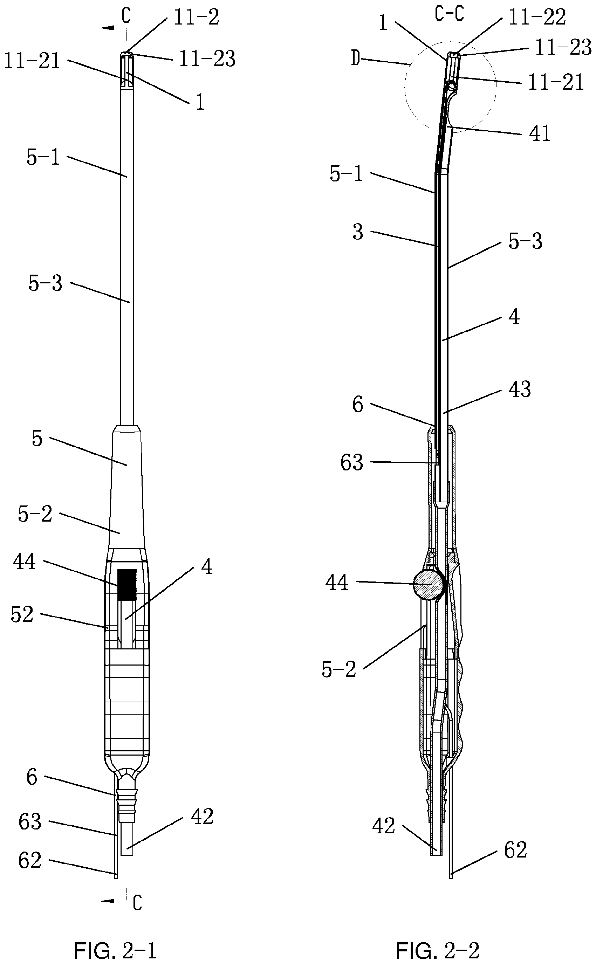

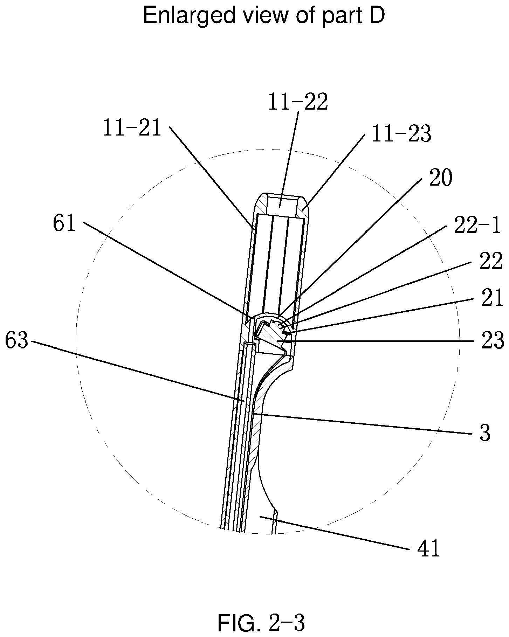

FIG. 2-3 is an enlarged view of part D in FIG. 2-2.

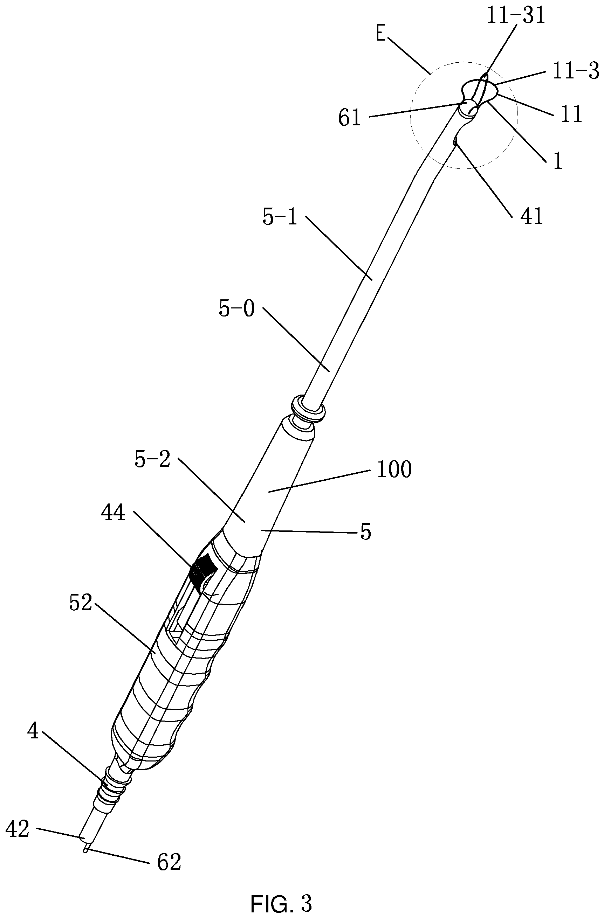

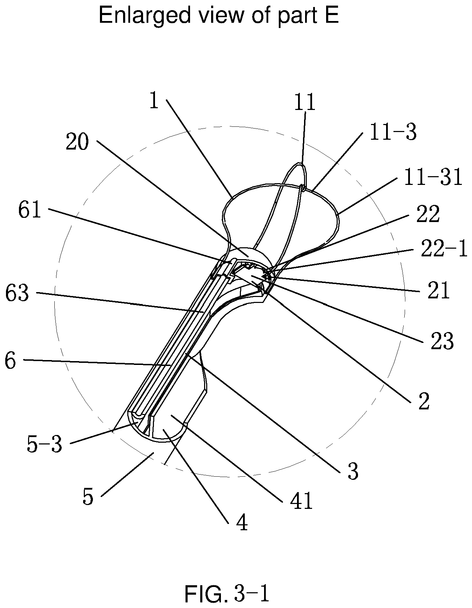

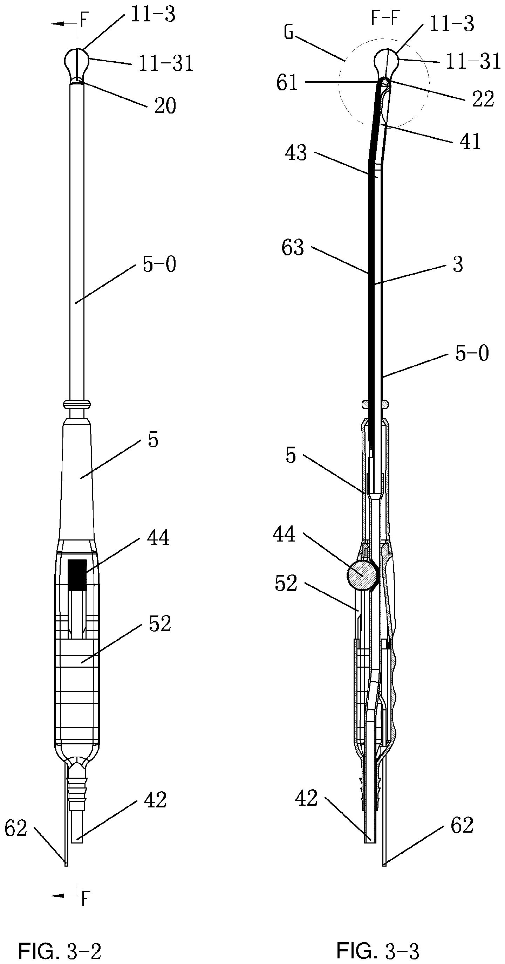

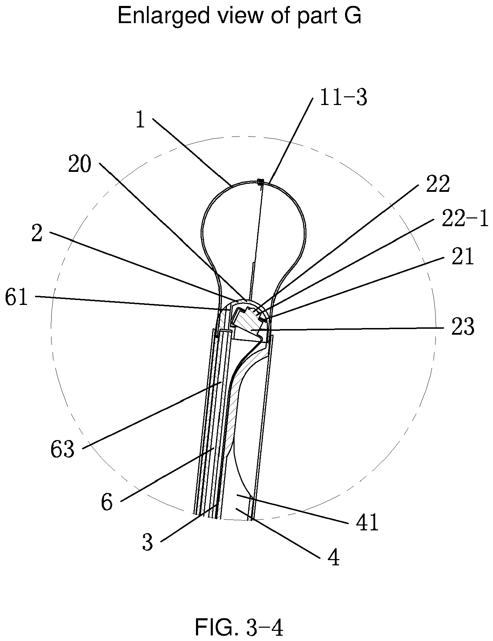

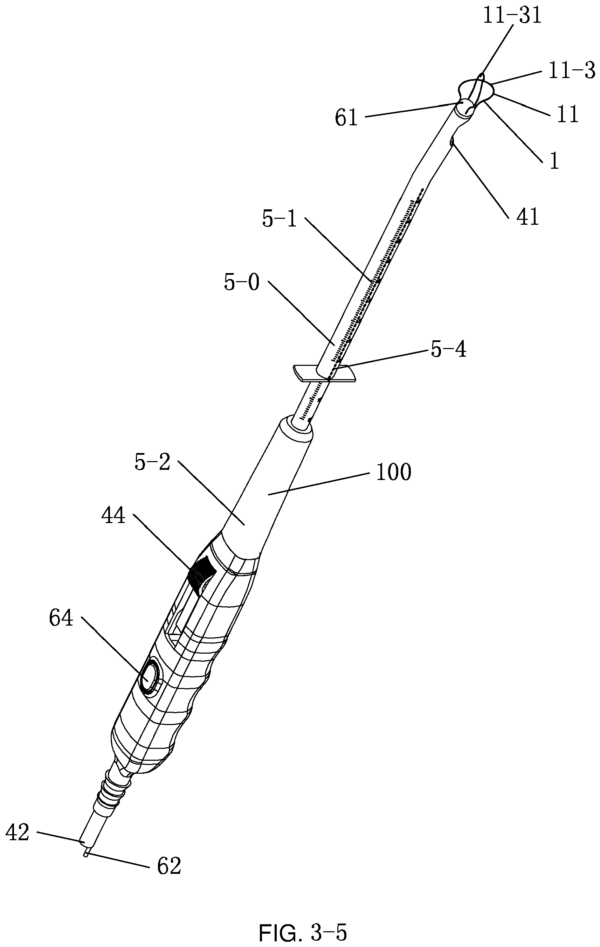

FIG. 3 is a three-dimensional schematic structural view of a direct-vision abortion uterine curettage device of the present invention with a curette mesh.
FIG. 3-1 is an enlarged view of part E in FIG. 3.
FIG. 3-2 is a front view of FIG. 3.
FIG. 3-3 is a cross-sectional view taken along line F-F in FIG. 3-2.
FIG. 3-4 is an enlarged view of part G in FIG. 3-3.
FIG. 3-5 is a three-dimensional schematic structural view of a direct-vision abortion uterine curettage device of the present invention with a tearable casing.

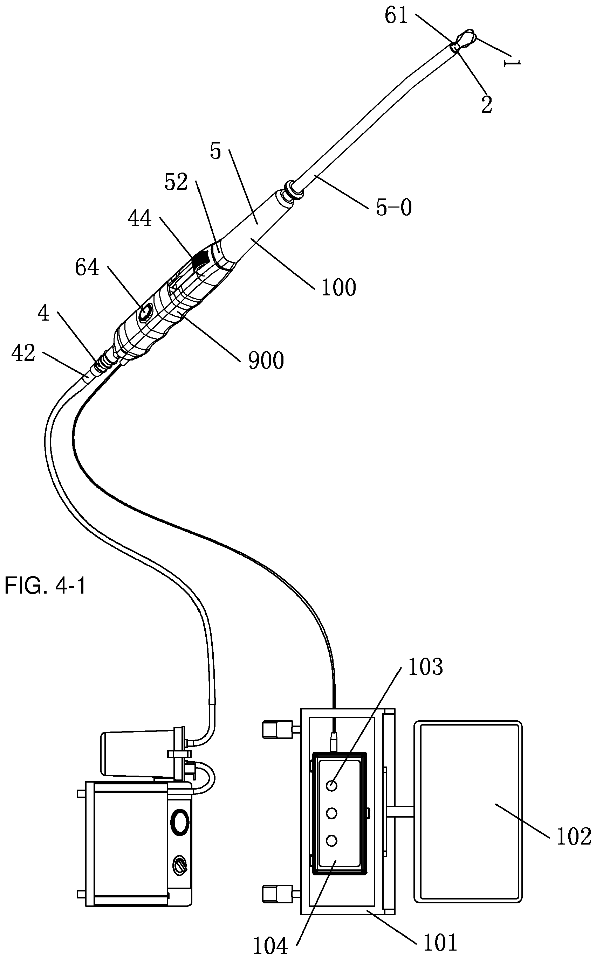

FIG. 4-1 is a schematic structural view of a direct-vision abortion uterine curettage system of the present invention.

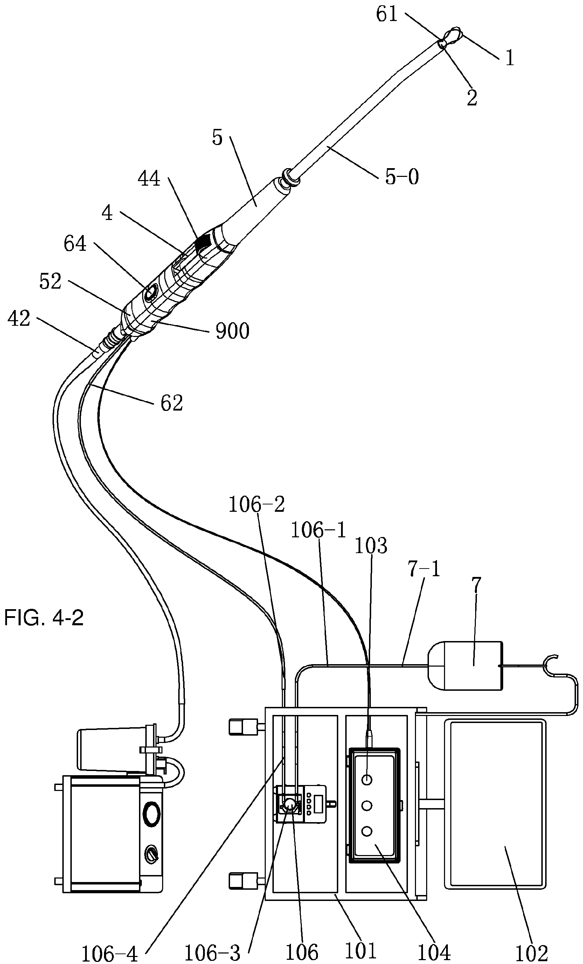

FIG. 4-2 is a schematic structural view of a direct-vision abortion uterine curettage system of the present invention with a flushing system.

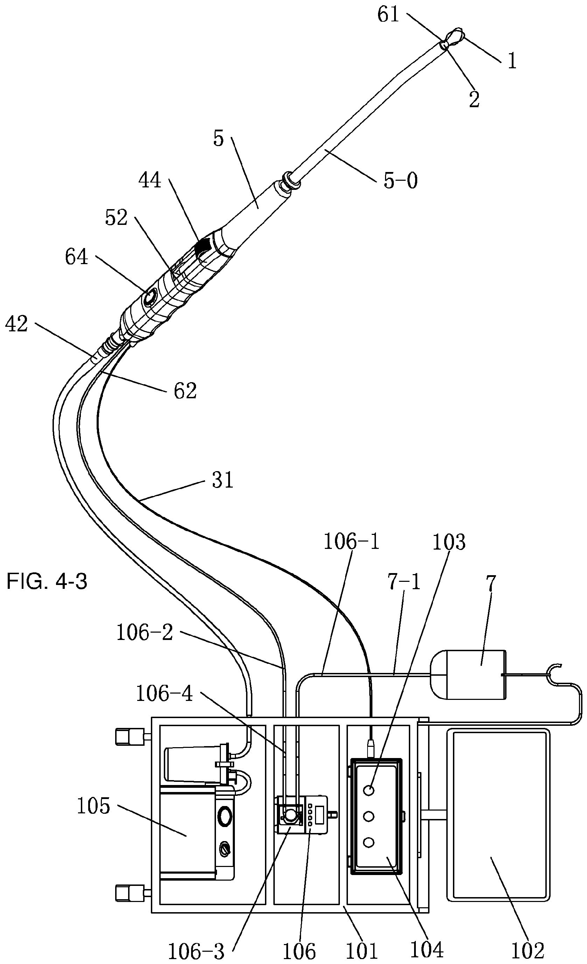

FIG. 4-3 is a schematic structural view of a direct-vision abortion uterine curettage system of the present invention with a negative pressure suction apparatus.

In the foregoing figures:
100 represents direct-vision abortion uterine curettage device of the present invention, and 900 represents direct-vision abortion uterine curettage system of the present invention.
101 represents housing, 102 represents display system, 103 represents image processing system, 104 represents power supply system, 105 represents negative pressure suction apparatus, and 106 represents flushing system.
1 represents uterine curettage mechanism, 2 represents observation mechanism, 3 represents circuit, 4 represents negative pressure suction mechanism, 5 represents operating rod, 6 represents flushing mechanism, 7 represents infusion bottle or bag, and 8 represents identification system.
11 represents diagnostic curette; 11-1 represents curette spoon, and 11-11 represents curette hook; 11-2 represents curette cup, 11-21 represents diagnostic curette strip, 11-22 represents through hole, and 11-23 represents curette table; 11-3 represents curette mesh, and 11-31 represents diagnostic curette wire.
20 represents protective cover, 21 represents illumination module, 22 represents lens module, and 23 represents signal processing module; and 22-1 represents camera.
31 represents circuit interface.
41 represents suction inlet, 42 represents suction outlet, 43 represents suction channel, and 44 represents negative pressure control switch.
5-0 represents sheath pipe, 5-1 represents front end, 5-2 represents rear end, 5-3 represents pipe body, and 5-4 represents casing; and 52 represents handle.
61 represents water outlet, 62 represents water inlet, 63 represents water inlet pipeline, and 64 represents flushing switch.
7-1 represents pipeline, and 81 represents graduated scale.
106-1 represents water inlet, 106-2 represents water outlet, 106-3 represents peristaltic pump, and 106-4 represents water pipe.

### DESCRIPTION OF EMBODIMENTS

### Embodiment 1: Direct-vision abortion uterine curettage device of the present invention with curette spoon

Referring to FIG. 1 to FIG. 1-4, the direct-vision abortion uterine curettage device of this embodiment includes a uterine curettage mechanism 1, an observation mechanism 2, a circuit 3, a negative pressure suction mechanism 4 and an operating rod 5.

The uterine curettage mechanism 1 includes at least one diagnostic curette 11, and the diagnostic curette 11 is arranged at a front end of the operating rod 5 and is in a visual field of the observation mechanism 2.

The observation mechanism 2 includes an illumination module 21, a lens module 22 and a signal processing module 23. Images and videos observed by the lens module 22 in a light field of the illumination module 21 are processed by the signal processing module 23, and then output by the circuit 3.

The negative pressure suction mechanism 4 includes a suction inlet 41, a suction outlet 42 and a suction channel 43. The suction inlet 41 is arranged at the front end of the operating rod 5; the suction outlet 42 of the negative pressure suction mechanism 4 is arranged at a rear end of the operating rod 5; and the suction channel 43 is arranged in the operating rod 5.

The rear end 5-2 of the operating rod 5 is provided with a handle 52, and the front end 5-1 of the operating rod 5 is provided with the uterine curettage mechanism 1 and the observation mechanism 2. The circuit 3 and the negative pressure suction mechanism 4 are located in a pipe body 5-3 of the operating rod 5.

Referring to FIG. 1, in this embodiment, the diagnostic curette 11 is a curette spoon 11-1. A front end of the curette spoon 11-1 is a blunt end, and a curette hook 11-11 of the curette spoon 11-1 is bent inward. The design of the blunt end may prevent the curette spoon 11-1 from accidentally puncturing the tissue at the front end of the curette spoon 11-1 during the forward moving process, and the clinical use process is safer. The inward bent curette hook 11-11 may conveniently remove the implanted embryo tissue.

In this embodiment, the curette spoon 11-1 is made of a transparent medical material. The transparent material may guarantee that the curette spoon 11-1 may not block an observation path of the camera 22-1, thereby guaranteeing that the tissue at the front end of the curette spoon 11-1 may also be observed by the observation mechanism 2, and guaranteeing the whole-process and overall-area observation of the operation procedure.

Referring to FIG. 1-2 and FIG. 1-3, in this embodiment, the lens module 22 includes a camera 22-1, and the camera 22-1 is arranged at the rear end of the diagnostic curette 11. The illumination system 21 is a light-emitting diode (LED) lamp arranged around the camera 22-1. The diagnostic curette 11 is in the visual field of the camera 22-1.

The observation mechanism 2 further includes a protective cover 20, and the protective cover 20 may prevent accidents such as short circuit of the observation mechanism 2 caused by fluids. The protective cover 20 of the observation mechanism 2 is provided with a coating. A hydrophobic coating may be selected, so that fluids such as watery blood may quickly condense into water droplets on the protective cover 20 and then slide off. A hydrophilic coating may also be selected, so that fluids such as watery blood may quickly form a transparent water film on a surface of the protective cover 20 without blocking the camera.

Referring to FIG. 1-2, in this embodiment, the direct-vision abortion uterine curettage device 100 further includes a flushing mechanism 6. A water outlet 61 of the flushing mechanism 6 is located at the front end of the operating rod 5; a water inlet 62 of the flushing mechanism 6 is located at the rear end of the operating rod 5; and a water inlet pipeline 63 of the flushing mechanism 6 is located in the pipe body 5-3 of the operating rod 5. The flushing mechanism 6 may flush the inside of the uterus during the operation procedure, and may flush the watery blood at the front end of the camera 22-1 in time to guarantee that the visual field of observation is kept clean. At the same time, the flushing mechanism 6 may also flush the diagnostic curette 11 and the uterus wall in time, especially may flush a surgical site in time, so as to observe whether the embryo tissue has been completely removed from the implantation position in time to guarantee the completeness of the embryo tissue during the abortion operation procedure and effectively avoid the condition of incomplete abortion, and the clinical operation procedure is safer.

In this embodiment, the direct-vision abortion uterine curettage device 100 further includes the flushing mechanism 6. Therefore, in this embodiment, the pipe body 5-3 of the operating rod 5 is a three-cavity pipe. The circuit 3 is arranged in the first cavity pipe, the second cavity pipe constitutes or is provided with the suction channel 43 of the negative pressure suction mechanism 4, and the third cavity pipe constitutes or is provided with the water inlet pipeline 63 of the flushing mechanism 6. The design of the three-cavity channel may separate a flushing channel from the suction channel while guaranteeing that the circuit 3 is isolated and protected, so as to better guarantee that no sucked tissue or fluid will return to the uterus during the flushing process, and the use process is more efficient and safer.

In practical applications, when the direct-vision abortion uterine curettage device 100 does not include the flushing mechanism 6, the pipe body 5-3 of the operating rod 5 may be designed into a double-cavity pipe. The circuit 3 is arranged in one cavity pipe, and the other cavity pipe constitutes or is provided with the suction channel 43 of the negative pressure suction mechanism 4. The design of the double-cavity channel avoids accidents such as short circuit and electric leakage, and at the same time, the structure is simpler.

Water sprayed from at least one water outlet 61 of the water outlets 61 of the flushing mechanism 6 of the direct-vision abortion uterine curettage device 100 may flush and clean the protective cover 20 of the observation mechanism 2, thereby guaranteeing the observation effect of the observation mechanism 2. Referring to FIG. 1-3, in this embodiment, the flushing mechanism 6 includes three water outlets 61. The first water outlet 61 is arranged close to the protective cover 20 and may flush the protective cover 20, thereby guaranteeing the observation effect of the observation mechanism 2. The second water outlet 61 is arranged close to the bottom of the curette hook 11-11 of the curette spoon 11-1 and may flush a curette surface of the curette hook 11-11, thereby guaranteeing the cleanliness of an inner surface of the curette hook 11-11. The third water outlet 61 is arranged at the front end of the curette spoon 11-1 and may flush an outer surface of the curette spoon 11-1 and surrounding operation areas, thereby observing whether the embryo tissue has been completely removed from the implantation position in time to guarantee the completeness of the embryo tissue during the abortion operation procedure and effectively avoid the condition of incomplete abortion.

In this embodiment, the flushing mechanism 6 is provided with a flushing switch 64 for controlling flushing water. The flushing switch 64 may start and stop the flushing mechanism 6, and may control the water volume and flushing pressure of the flushing water. During the clinical use process, clinicians may start or stop the flushing mechanism 6 according to actual needs of the operation procedure, and select the appropriate volume and pressure of the flushing water according to needs, and the clinical use process is more convenient.

The flushing switch 64 is arranged on the handle 52 of the operating rod 5. By arranging the flushing switch 64 on the handle 52, the medical staff may realize one-handed operation during clinical use, and the use process is more convenient.

The negative pressure suction mechanism 4 is provided with a negative pressure control switch 44 capable of controlling a negative pressure state. The negative pressure control switch 44 may start or stop the negative pressure suction mechanism 4, and may adjust the negative pressure suction force of the negative pressure suction mechanism 4 according to the volume of the peeled tissue, watery blood and tissue fluids in the uterus during the operation procedure. During clinical use, doctors may conveniently adjust the needed negative pressure suction force, so as to avoid that the peeled tissue may not be sucked out in time due to a too small negative pressure suction force, and avoid accidental injuries to the endometrium and other tissues due to a too large negative pressure suction force.

The negative pressure control switch 44 is arranged on the handle 52 of the operating rod 5. By arranging the negative pressure control switch on the handle 52, the medical staff may realize one-handed operation during clinical use, and the use process is more convenient.

Referring to FIG. 1-4, the circuit 3 is provided with an electrical interface 31 connected with a host.

The direct-vision abortion uterine curettage device 100 further includes an identification system 8. The identification system 8 may prompt the depth of the operating rod 5 into the human body. In this embodiment, the identification system 8 is a graduated scale 81 arranged outside the operating rod 5. Of course, in practical applications, those skilled in the art may also design the identification system 8 into other identification methods which are not specifically illustrated by the applicant here one by one, but do not depart from the protection scope of the present application.

During a clinical operation, the handle 52 is held, under the condition of direct vision, the direct-vision abortion uterine curettage device 100 enters the uterus through the cervix along the vagina, the implantation position of the embryo tissue is found through the camera 22-1, and then, the curette hook 11-11 of the curette spoon 11-1 is used to remove the embryo tissue from the uterus wall. The removed embryo tissue is sucked from the suction inlet 41 under the negative pressure suction action of the negative pressure suction mechanism 4, and is discharged out of the body through the suction outlet 42 after passing through the suction channel 43. During the suction process, doctors may adjust the magnitude of the negative pressure suction force through the control switch 44 arranged on the handle 52 according to the size of the removed tissue and the volume of watery blood. During the operation procedure, the flushing mechanism 6 may be used for flushing. On the one hand, the protective cover 20 of the observation mechanism 2 may be flushed in time to keep the real-time observation effect of the observation mechanism 2 good and stable; and on the other hand, the curette spoon 11-1 and the operation area may be flushed, thereby observing whether the embryo tissue has been completely removed from the implantation position in time to guarantee the completeness of the embryo tissue during the abortion operation procedure and effectively avoid the condition of incomplete abortion, and the clinical operation procedure is safer.

In this embodiment, the technical solution of the camera 22-1 being mounted at the rear may guarantee that the diagnostic curette 11 is completely within a range of the visual field of the camera 22-1, and since an observation angle of the camera 22-1 faces the front of an operation area, there will be no observation blind areas during the observation process. As a result, there will be no accidental injuries to the uterus and uterine appendages caused by the failure to observe clearly in time. At the same time, since the diagnostic curette 11 is located in front of the camera 22-1, there will be no operation blind spots due to the diagnostic curette 11 being blocked by the height of the lens module 22 itself. The embryo tissue implanted at any position of the uterus, especially near the oviduct orifice, may be completely removed, i.e. the diagnostic curette 11 may also easily remove the embryo tissue at the implantation position of the uterine fundus, and the condition of incomplete abortion may be avoided during the operation procedure. Moreover, since the removal is performed under direct vision, an accidental injury to the uterine fundus due to an excessive surgical action may be avoided, the injury to the uterine fundus may be well avoided, and serious medical accidents such as perforation of uterus may be avoided. The clinical operation procedure is very safe and efficient.

### Embodiment 2: Direct-vision abortion uterine curettage device of the present invention with curette cup

Referring to FIG. 2 to FIG. 2-3, the difference between this embodiment and Embodiment 1 is that: in this embodiment, the diagnostic curette 11 is a curette cup 11-2.

A front end of the curette cup 11-2 is a blunt end, and at least one diagnostic curette strip 11-21 is arranged around the blunt end. The design of the blunt end may guarantee that the curette cup 11-2 may not accidentally puncture the tissue at the front end of the curette cup 11-2 during the forward moving process. During clinical use, the diagnostic curette strip 11-21 may conveniently remove the implanted embryo tissue from the uterus.

Referring to FIG. 2-2 and FIG. 2-3, in this embodiment, a curette table 11-23 provided with a through hole 11-22 is arranged at the front end of the curette cup 11-2. During a clinical operation, especially when the implantation position of the embryo tissue is at the uterine fundus, as long as the curette cup 11-2 is rotated, the curette table 11-23 also rotates, and then, the embryo tissue may be removed from the uterine fundus. During clinical use, the curette cup may adapt to different embryo implantation positions.

The curette cup 11-2 is made of a transparent medical material. The transparent material may guarantee that the curette cup 11-2 may not block the observation path of the camera 22-1, thereby guaranteeing that the tissue at the front end of the curette cup 11-2 may also be observed by the observation mechanism 2, and guaranteeing the whole-process and overall-area observation of the operation procedure.

In this embodiment, the diagnostic curette 11 is the curette cup 11-2. The curette table 11-23 may be formed at the bottom of the curette cup 11-2, and the curette table 11-23 is located at the most front end of the direct-vision abortion uterine curettage device 100, so that the curette table 11-23 may be attached to the uterus to remove the embryo tissue, and may also completely remove the embryo tissue even at the implantation position of the uterine fundus conveniently.

### Embodiment 3: Direct-vision abortion uterine curettage device of the present invention with curette mesh

Referring to FIG. 3 to FIG. 3-4, the difference between this embodiment and Embodiment 1 is that: in this embodiment, the diagnostic curette 11 is a curette mesh 11-3.

In this embodiment, the diagnostic curette 11 of the uterine curettage mechanism 1 is a curette mesh 11-3. Through the camera 22-1, the condition behind the curette mesh 11-3 may be directly observed from mesh gaps, and even if the curette mesh 11-3 is not made of a transparent material, the whole-process observation of the operation procedure and the panoramic observation of the operation area may be realized.

Referring to FIG. 3, in this embodiment, the curette mesh 11-3 of the diagnostic curette 11 of the uterine curettage mechanism 1 may be compressed in a sheath pipe 5-0, and after the constraint of the sheath pipe 5-0 is removed backward, the curette mesh 11-3 may be completely restored or basically restored to a shape before compression.

Referring to FIG. 3-1, the curette mesh 11-3 includes at least one diagnostic curette wire 11-31. In this embodiment, the curette mesh 11-3 is formed by winding two diagnostic curette wires 11-31 and has a cross shape, and the top end is wound by the diagnostic curette wires 11-31 to form an annular platform.

In practical applications, the curette mesh 11-3 may be formed by winding one diagnostic curette wire 11-31 or superimposing a plurality of diagnostic curette wires 11-31 after being wound. Moreover, the curette mesh 11-3 may be manufactured in any shapes which are not illustrated by the applicant here one by one, but do not depart from the protection scope of the present application.

The diagnostic curette 11 of the uterine curettage mechanism 1 may also be a woven mesh made of a wire material. The design of the soft woven mesh makes the force applied during the implanted embryo removing process more gentle and even, so that the injury to the uterus is less.

The curette mesh 11-3 is made of a medical elastic material. The curette mesh 11-3 may guarantee the removing effect, and at the same time, during the forward moving process, the elastic action of the medical elastic material may play a good buffering effect, so that the curette mesh 11-3 will not cause an accidental injury to the uterus due to an excessively fast moving speed.

The curette mesh 11-3 is made of medical elastic stainless steel or medical titanium-nickel shape memory alloy. Here, the applicant only lists the above two medical metallic elastic materials. In practical applications, those skilled in the art may also select different medical elastic materials for manufacturing according to needs, and all the medical elastic materials do not depart from the protection scope of the present application.

The curette mesh 11-3 is made of a medical transparent elastic polymer material. The curette mesh 11-3 may also be made of the medical transparent elastic polymer material, so that the observation path of the camera 22-1 may not be blocked while guaranteeing elastic buffering, and the observation effect is better.

In order to guarantee the safety in clinical use of the direct-vision abortion uterine curettage device 100, the direct-vision abortion uterine curettage device 100 adopts a disposable design. The disposable use may be realized through a control program design, such as setting a use time, or may be realized through a structural design. Referring to FIG. 3-5, for example, a tearable casing 5-4 may be arranged outside the direct-vision abortion uterine curettage device 100. After the direct-vision abortion uterine curettage device 100 enters the uterus, the casing 5-4 needs to be torn off, and the curette mesh 11-3 is released. After the operation is completed, when the direct-vision abortion uterine curettage device 100 is taken from the body, since the curette mesh 11-3 has been released, the curette mesh 11-3 may not enter the body again for repeated use, so as to realize disposable use of the direct-vision abortion uterine curettage device 100.

In this embodiment, the curette mesh 11-3 is made of an elastic material, the curette mesh 11-3 may guarantee the removing effect, and at the same time, during the forward moving process, the elastic action of the medical elastic material may play a good buffering effect, so that the curette mesh 11-3 will not cause an accidental injury to the uterus due to an excessively fast moving speed. The use process is safer.

### Embodiment 4: Direct-vision abortion uterine curettage system of the present invention

Referring to FIG. 4-1, the direct-vision abortion uterine curettage system of this embodiment includes the direct-vision abortion uterine curettage device in Embodiment 3.

In this embodiment, the direct-vision abortion uterine curettage system 900 includes the direct-vision abortion uterine curettage device 100, a housing 101, a display system 102, an image processing system 103 and a power supply system 104; the display system 102 is arranged on the housing 101; the image processing system 103 and the power supply system 104 are installed in the housing 101; the display system 102, the image processing system 103 and the power supply system 104 are connected together through the circuit 3; and the suction outlet 42 of the negative pressure suction mechanism 4 of the direct-vision abortion uterine curettage device 100 is connected with a negative pressure suction apparatus of a hospital or a negative pressure pipe of a medical special negative pressure source.

During clinical use, the suction outlet 42 is connected with the negative pressure suction apparatus of the hospital or the negative pressure pipe of the medical special negative pressure source; the power supply system 104 is turned on, the direct-vision abortion uterine curettage system 900 starts to work; the observation mechanism 2 is started, and video signals collected by the observation mechanism 2 are processed by the image processing system 103 and then may be transmitted to the display system 102 in a wired or wireless manner and displayed on the display system 102. Under the observation of the camera 22-1, the direct-vision abortion uterine curettage device 100 enters the uterus through the cervix along the vagina to find the implanted embryo tissue, then the diagnostic curette 11 of the uterine curettage mechanism 1 is used to peel the implanted embryo tissue from the uterus; the negative pressure suction apparatus or the medical special negative pressure source is turned on, and the peeled embryo tissue and accompanying watery blood are sucked through the suction outlet 42 and discharged out of the body until the embryo tissue is completely peeled and sucked and discharged out of the body, thereby completing the abortion operation.

The direct-vision abortion uterine curettage system of this embodiment may visually display the whole processes of the direct-vision abortion uterine curettage device 100 entering the uterus and the removal of the embryo tissue on the display system 102, and there will be no incomplete abortion during the operation procedure. Moreover, since the removal is performed under direct vision, an accidental injury to the uterine fundus due to an excessive surgical action may be avoided, the injury to the uterine fundus may be well avoided, and serious medical accidents such as perforation of uterus may be avoided. The clinical operation procedure is very safe and efficient.

### Embodiment 5: Direct-vision abortion uterine curettage system of the present invention with flushing system

Referring to FIG. 4-2, the difference between the direct-vision abortion uterine curettage system of this embodiment and Embodiment 4 is that: in this embodiment, the direct-vision abortion uterine curettage system further includes a flushing system 106.

In this embodiment, the direct-vision abortion uterine curettage system 900 includes the direct-vision abortion uterine curettage device 100, a housing 101, a display system 102, an image processing system 103, a power supply system 104 and a flushing system 106; the display system 102 is arranged on the housing 101; the image processing system 103, the power supply system 104 and the flushing system 106 are installed in the housing 101; the display system 102, the image processing system 103, the power supply system 104 and the flushing system 106 are connected together through the circuit 3; the suction outlet 42 of the negative pressure suction mechanism 4 of the direct-vision abortion uterine curettage device 100 is connected with a negative pressure suction apparatus of a hospital or a negative pressure pipe of a medical special negative pressure source; and a water inlet 106-1 of the flushing system 106 is connected with an infusion bottle or bag 7 through a water pipe 106-4, and a water outlet 106-2 of the flushing system 106 is connected with the water inlet 62 of the flushing mechanism 6 of the direct-vision abortion uterine curettage device 100 through the water pipe 106-4.

In this embodiment, the flushing system 106 is driven by a peristaltic pump 106-3. The peristaltic pump 106-3 may more accurately control the flow rate and speed of water intake. Of course, in practical applications, those skilled in the art may also use different driving devices to drive the flushing system 106, which does not depart from the protection scope of the present application.

The direct-vision abortion uterine curettage system of this embodiment includes the flushing system 106, and the flushing system 106 may flush the direct-vision abortion uterine curettage device 100 and surrounding tissues in time during the operation procedure, so as to flush a surgical site in time while guaranteeing that the visual field of observation is kept clean, thereby observing whether the embryo tissue has been completely removed from the implantation position in time to guarantee the completeness of the embryo tissue during the abortion operation procedure and effectively avoid the condition of incomplete abortion. The clinical operation procedure is safer.

### Embodiment 6: Direct-vision abortion uterine curettage system of the present invention with negative pressure source

Referring to FIG. 4-3, the difference between the direct-vision abortion uterine curettage system of this embodiment and Embodiment 5 is that: in this embodiment, the direct-vision abortion uterine curettage system further includes a negative pressure suction apparatus 105.

In this embodiment, the direct-vision abortion uterine curettage system 900 includes the direct-vision abortion uterine curettage device 100, a housing 101, a display system 102, an image processing system 103, a power supply system 104, a negative pressure suction apparatus 105 and a flushing system 106; the display system 102 is arranged on the housing 101; the image processing system 103, the power supply system 104, the negative pressure suction apparatus 105 and the flushing system 106 are installed in the housing 101; the display system 102, the image processing system 103, the power supply system 104, the negative pressure suction apparatus 105 and the flushing system 106 are connected together through the circuit 3; the suction outlet 42 of the negative pressure suction mechanism 4 of the direct-vision abortion uterine curettage device 100 is connected with the negative pressure suction apparatus 105; and a water inlet 106-1 of the flushing system 106 is connected with an infusion bottle or bag 7 through a pipeline 7-1, and a water outlet 106-2 of the flushing system 106 is connected with the water inlet 62 of the flushing mechanism 6 through the pipeline 7-1.

In this embodiment, the direct-vision abortion uterine curettage system 900 includes the negative pressure suction apparatus 105 and the flushing system 106, a negative pressure suction function and a flushing function are integrated into one device, and the whole operation procedure of direct-vision abortion may be completed only by the one device without relying on external negative pressure sources and external flushing systems, thereby greatly reducing the limitation and influence of the external environment on the operation procedure. The direct-vision abortion uterine curettage system has a very wide scope of application.

It should be noted that the structure disclosed and described in this specification may be replaced with another structure with the same effect. In addition, the embodiments described in the present invention are not the only structure of implementing the present invention. Although exemplary embodiments of the present invention have been introduced and described in this specification, it should be understood by a person skilled in the art that the embodiments are merely described by way of example, and a person skilled in the art may make various changes, improvements, and replacements without departing from the present invention. Therefore, the protection scope of the present invention should be defined in accordance with the spirit and scope of the claims appended to the present invention.

## Claims

1. A direct-vision abortion uterine curettage device and system, wherein the direct-vision abortion uterine curettage device (100) comprises a uterine curettage mechanism (1), an observation mechanism (2), a circuit (3), a negative pressure suction mechanism (4) and an operating rod (5);
A. the uterine curettage mechanism (1) comprises at least one diagnostic curette (11), and the diagnostic curette (11) is arranged at a front end of the operating rod (5) and is in a visual field of the observation mechanism (2);
B. the observation mechanism (2) comprises an illumination module (21), a lens module (22) and a signal processing module (23), and images and videos observed by the lens module (22) in a light field of the illumination module (21) are processed by the signal processing module (23), and then output by the circuit (3);
C. the negative pressure suction mechanism (4) comprises a suction inlet (41), a suction outlet (42) and a suction channel (43), the suction inlet (41) is arranged at the front end of the operating rod (5), the suction outlet (42) of the negative pressure suction mechanism (4) is arranged at a rear end of the operating rod (5), and the suction channel (43) is arranged in the operating rod (5); and
D. the rear end (5-2) of the operating rod (5) is provided with a handle (52), the front end (5-1) of the operating rod (5) is provided with the uterine curettage mechanism (1) and the observation mechanism (2), and the circuit (3) and the negative pressure suction mechanism (4) are located in a pipe body (5-3) of the operating rod (5).

2. The direct-vision abortion uterine curettage device according to claim 1, wherein a camera (22-1) of the lens module (22) is arranged at a rear end of the diagnostic curette (11), and the diagnostic curette (11) is in a visual field of the camera (22-1).

3. The direct-vision abortion uterine curettage device according to claim 1, wherein the direct-vision abortion uterine curettage device (100) further comprises a flushing mechanism (6), a water outlet (61) of the flushing mechanism (6) is located at the front end of the operating rod (5), a water inlet (62) of the flushing mechanism (6) is located at the rear end of the operating rod (5), and a water inlet pipeline (63) of the flushing mechanism (6) is located in the pipe body (5-3) of the operating rod (5).

4. The direct-vision abortion uterine curettage device according to claim 1, wherein the pipe body (5-3) of the operating rod (5) is a double-cavity pipe, the circuit (3) is arranged in one cavity pipe, and the other cavity pipe constitutes or is provided with the suction channel (43) of the negative pressure suction mechanism (4).

5. The direct-vision abortion uterine curettage device according to claim 3, wherein the pipe body (5-3) of the operating rod (5) is a three-cavity pipe, the circuit (3) is arranged in the first cavity pipe, the second cavity pipe constitutes or is provided with the suction channel (43) of the negative pressure suction mechanism (4), and the third cavity pipe constitutes or is provided with the water inlet pipeline (63) of the flushing mechanism (6).

6. The direct-vision abortion uterine curettage device according to claim 1, wherein water sprayed from at least one water outlet (61) of the water outlets (61) of the flushing mechanism (6) of the direct-vision abortion uterine curettage device (100) may flush and clean a protective cover (20) of the observation mechanism (2).

7. The direct-vision abortion uterine curettage device according to claim 1, wherein the flushing mechanism (6) of the direct-vision abortion uterine curettage device (100) is provided with a flushing switch (64) for controlling flushing water.

8. The direct-vision abortion uterine curettage device according to claim 7, wherein the flushing switch (64) is arranged on the handle (52) of the operating rod (5).

9. The direct-vision abortion uterine curettage device according to claim 1, wherein the negative pressure suction mechanism (4) is provided with a negative pressure control switch (44) capable of controlling a negative pressure state.

10. The direct-vision abortion uterine curettage device according to claim 9, wherein the negative pressure control switch (44) is arranged on the handle (52) of the operating rod (5).

11. The direct-vision abortion uterine curettage device according to claim 1, wherein the diagnostic curette (11) of the uterine curettage mechanism (1) is a curette spoon (11-1).

12. The direct-vision abortion uterine curettage device according to claim 11, wherein a front end of the curette spoon (11-1) is a blunt end, and a curette hook (11-11) of the curette spoon (11-1) is bent inward.

13. The direct-vision abortion uterine curettage device according to claim 11, wherein the curette spoon (11-1) is made of a transparent medical material.

14. The direct-vision abortion uterine curettage device according to claim 1, wherein the diagnostic curette (11) of the uterine curettage mechanism (1) is a curette cup (11-2).

15. The direct-vision abortion uterine curettage device according to claim 14, wherein a front end of the curette cup (11-2) is a blunt end, and at least one diagnostic curette strip (11-21) is arranged around the blunt end.

16. The direct-vision abortion uterine curettage device according to claim 14, wherein the curette cup (11-2) is made of a transparent medical material.

17. The direct-vision abortion uterine curettage device according to claim 1, wherein the diagnostic curette (11) of the uterine curettage mechanism (1) is a curette mesh (11-3).

18. The direct-vision abortion uterine curettage device according to claim 17, wherein the curette mesh (11-3) of the diagnostic curette (11) of the uterine curettage mechanism (1) may be compressed in a sheath pipe (5-0).

19. The direct-vision abortion uterine curettage device according to claim 17, wherein the curette mesh (11-3) of the diagnostic curette (11) of the uterine curettage mechanism (1) may be compressed in a sheath pipe (5-0), and after the constraint of the sheath pipe (5-0) is removed backward, the curette mesh (11-3) may be completely restored or basically restored to a shape before compression.

20. The direct-vision abortion uterine curettage device according to claim 17, wherein the curette mesh (11-3) at least comprises one diagnostic curette wire (11-31).

21. The direct-vision abortion uterine curettage device according to claim 17, wherein the diagnostic curette (11) of the uterine curettage mechanism (1) is a woven mesh made of a wire material.

22. The direct-vision abortion uterine curettage device according to claim 17, wherein the curette mesh (11-3) is made of a medical elastic material.

23. The direct-vision abortion uterine curettage device according to claim 17, wherein the curette mesh (11-3) is made of medical elastic stainless steel or medical titanium-nickel shape memory alloy.

24. The direct-vision abortion uterine curettage device according to claim 17, wherein the curette mesh (11-3) is made of a medical transparent elastic polymer material.

25. The direct-vision abortion uterine curettage device according to claim 1, wherein a protective cover (20) of the observation mechanism (2) is provided with a coating.

26. The direct-vision abortion uterine curettage device according to claim 1, wherein the circuit (3) is provided with an electrical interface (31) connected with a host.

27. The direct-vision abortion uterine curettage device according to claim 1, wherein the direct-vision abortion uterine curettage device (100) further comprises an identification system (8).

28. A direct-vision abortion uterine curettage system, wherein the direct-vision abortion uterine curettage system (900) comprises the direct-vision abortion uterine curettage device (100) according to claim 1.

29. The direct-vision abortion uterine curettage system according to claim 28, wherein the direct-vision abortion uterine curettage system (900) comprises the direct-vision abortion uterine curettage device (100), a housing (101), a display system (102), an image processing system (103) and a power supply system (104); the display system (102) is arranged on the housing (101); the image processing system (103) and the power supply system (104) are installed in the housing (101); the display system (102), the image processing system (103) and the power supply system (104) are connected together through the circuit (3); and the suction outlet (42) of the negative pressure suction mechanism (4) of the direct-vision abortion uterine curettage device (100) is connected with a negative pressure suction apparatus of a hospital or a negative pressure pipe of a medical special negative pressure source.

30. The direct-vision abortion uterine curettage system according to claim 28, wherein the direct-vision abortion uterine curettage system (900) comprises the direct-vision abortion uterine curettage device (100), a housing (101), a display system (102), an image processing system (103), a power supply system (104) and a negative pressure suction apparatus (105); the display system (102) is arranged on the housing (101); the image processing system (103), the power supply system (104) and the negative pressure suction apparatus (105) are installed in the housing (101); the display system (102), the image processing system (103), the power supply system (104) and the negative pressure suction apparatus (105) are connected together through the circuit (3); and the suction outlet (42) of the negative pressure suction mechanism (4) of the direct-vision abortion uterine curettage device (100) is connected with the negative pressure suction apparatus (105).

31. The direct-vision abortion uterine curettage system according to claim 28, wherein the direct-vision abortion uterine curettage system (900) comprises the direct-vision abortion uterine curettage device (100), a housing (101), a display system (102), an image processing system (103), a power supply system (104) and a flushing system (106); the display system (102) is arranged on the housing (101); the image processing system (103), the power supply system (104) and the flushing system (106) are installed in the housing (101); the display system (102), the image processing system (103), the power supply system (104) and the flushing system (106) are connected together through the circuit (3); the suction outlet (42) of the negative pressure suction mechanism (4) of the direct-vision abortion uterine curettage device (100) is connected with a negative pressure suction apparatus of a hospital or a negative pressure pipe of a medical special negative pressure source; and a water inlet (106-1) of the flushing system (106) is connected with an infusion bottle or bag (7) through a water pipe (106-4), and a water outlet (106-2) of the flushing system (106) is connected with the water inlet (62) of the flushing mechanism (6) of the direct-vision abortion uterine curettage device (100) through the water pipe (106-4).

32. The direct-vision abortion uterine curettage system according to claim 28, wherein the direct-vision abortion uterine curettage system (900) comprises the direct-vision abortion uterine curettage device (100), a housing (101), a display system (102), an image processing system (103), a power supply system (104), a negative pressure suction apparatus (105) and a flushing system (106); the display system (102) is arranged on the housing (101); the image processing system (103), the power supply system (104), the negative pressure suction apparatus (105) and the flushing system (106) are installed in the housing (101); the display system (102), the image processing system (103), the power supply system (104), the negative pressure suction apparatus (105) and the flushing system (106) are connected together through the circuit (3); the suction outlet (42) of the negative pressure suction mechanism (4) of the direct-vision abortion uterine curettage device (100) is connected with the negative pressure suction apparatus (105); and a water inlet (106-1) of the flushing system (106) is connected with an infusion bottle or bag (7) through a pipeline (7-1), and a water outlet (106-2) of the flushing system (106) is connected with the water inlet (62) of the flushing mechanism (6) through the pipeline (7-1).

33. The direct-vision abortion uterine curettage system according to claim 32, wherein the flushing system (106) is driven by a peristaltic pump (106-3).
